# EUROPEAN PATENT APPLICATION

(11) **EP 4 484 083 A1**
(43) Date of publication of application: **01.01.2025**
(21) Application number: 23759635.8
(22) Date of filing: 03.02.2023
(51) Int. Cl.: B25J 18/06

(54) **CONTINUUM ROBOT SYSTEM, CONTROL METHOD THEREFOR, AND PROGRAM**

(30) Priority: 25.02.2022 JP 2022028144
(71) Applicant: CANON KABUSHIKI KAISHA, Tokyo 146-8501 (JP)
(72) Inventor: SATO, Kei, Tokyo 146-8501 (JP); IKEDA, Yoshimichi, Tokyo 146-8501 (JP)
(74) Representative: Canon Europe Limited
(86) International application number: PCT/JP2023/003506
(87) International publication number: WO 2023/162629

(57) **Abstract**

A continuum robot system includes: a continuum robot 1 including a bendable body 11 configured to bend via a plurality of linear members W, a first drive source M that moves the linear members W, and a tensile force detector 21cf that detects tensile forces that have occurred in the linear members W; a support base 2 including a moving stage 2a to which the continuum robot 1 is attached, and a second drive source 31 that slides the moving stage 2a; and a control apparatus 3 that controls, in a case where the tensile forces of the plurality of linear members W detected by the tensile force detector 21cf are tensile forces in a same direction and at least one of the tensile forces exceeds a predetermined value when the second drive source 31 is driven in a direction in which the bendable body 11 is inserted into a target, the second drive source 31 such that the second drive source 31 is stopped or driven in an opposite direction.

## Description

### Technical Field

The present invention relates to a continuum robot system, a method for controlling the same, and a program.

### Background Art

A medical device including a continuum robot is used. PTL 1 discloses a configuration in which a medical tool including a manipulator detects a tensile force that has occurred in a drive wire, and causes a tip flexing part to be in a flexed state along a channel member by controlling an amount by which the drive wire is driven by a drive motor such that the tensile force is in a target control range set in advance. Thus, in a case where the manipulator is inserted through the channel member and sent to a site for use, it is possible to easily perform the insertion operation.

### Citation List

### Patent Literature

PTL 1: Japanese Patent No. 6169049

### Summary of Invention

### Technical Problem

Since an organ that is in a human body and is treated as a target is complex, a distal end of a bendable body of a continuum robot may come into contact with an inner wall of the organ almost from the front when the bendable body of the continuum robot is inserted in the target. When the bendable body comes into contact with the inner wall of the organ, the bendable body may not be released from the contact only by controlling the flexing of the bendable body based on the tensile force as in PTL 1. When such a situation occurs, it is difficult to further insert the bendable body in the target.

The present invention has been made in view of the above-described circumstances, and aims at easily releasing a bendable body of a continuum robot from contact when the bendable body of the continuum robot is inserted in a target.

### Solution to Problem

A continuum robot system according to the present invention includes: a continuum robot including a bendable body configured to bend via a plurality of linear members, a first drive source that moves the linear members, and a tensile force detector that detects tensile forces that have occurred in the linear members; a support base including a moving stage to which the continuum robot is attached, and a second drive source that slides the moving stage; and control means that controls, in a case where the tensile forces of the plurality of linear members detected by the tensile force detector are all tensile forces in a same direction and at least one of the tensile forces exceeds a predetermined value when the second drive source is driven in a direction in which the bendable body is inserted into a target, the second drive source such that the second drive source is stopped or driven in an opposite direction. Advantageous Effects of Invention

According to the present invention, it is possible to easily release a bendable body of a continuum robot from contact when the bendable body of the continuum robot is inserted in a target.

### Brief Description of Drawings

[Fig. 1] Fig. 1 is an overall view of a medical system.
[Fig. 2] Fig. 2 is a perspective view illustrating a medical device and a support base.
[Fig. 3] Fig. 3 is an explanatory diagram of the support base.
[Fig. 4A] Fig. 4A is an explanatory diagram of a catheter.
[Fig. 4B] Fig. 4B is an explanatory diagram of the catheter.
[Fig. 5A] Fig. 5A is an explanatory diagram of a base unit and a wire drive section.
[Fig. 5B] Fig. 5B is an explanatory diagram of the base unit and the wire drive section.
[Fig. 6A] Fig. 6A is an explanatory diagram of the wire drive section, a coupling device, and a bending drive section.
[Fig. 6B] Fig. 6B is an explanatory diagram of the wire drive section, the coupling device, and the bending drive section.
[Fig. 7] is an explanatory diagram of a force sensor.
[Fig. 8A] is a diagram explaining coupling of a catheter unit and the base unit.
[Fig. 8B] is a diagram explaining the coupling of the catheter unit and the base unit.
[Fig. 9] is an exploded view explaining the coupling of the catheter unit and the base unit.
[Fig. 10] is a diagram explaining fixation of a drive wire by a coupling part.
[Fig. 11] is a diagram explaining the fixation of the drive wire by the coupling part.
[Fig. 12] is a diagram explaining the fixation of the drive wire by the coupling part.
[Fig. 13] is a diagram explaining the fixation of the drive wire by the coupling part.
[Fig. 14] is a diagram explaining the fixation of the drive wire by the coupling part.
[Fig. 15] is a flowchart illustrating a control process for releasing from contact by a control apparatus in Example 1.
[Fig. 16] is a flowchart illustrating a process of back draft control of a moving motor in Example 1.
[Fig. 17] is a flowchart illustrating a data acquisition process for Follow The Leader control.
[Fig. 18] Fig. 18 is a flowchart illustrating a process of Follow The Leader control for reverse rotation.
[Fig. 19] Fig. 19 is a flowchart illustrating a process of the back draft control of the moving motor in Example 2.
[Fig. 20] Fig. 20 is a diagram illustrating an example of a hardware configuration.

### Description of Embodiments

Hereinafter, Examples of the present invention will be described with reference to the drawings. Dimensions, materials, shapes, arrangements, and the like of constituent components described in Examples should be changed as appropriate according to configurations of apparatuses, various conditions, and the like to which the present invention is applied.

### [Example 1]

### <Medical System and Medical Device>

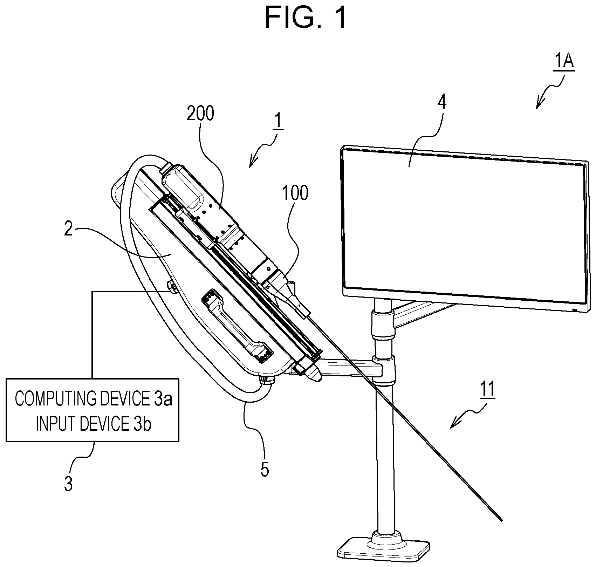

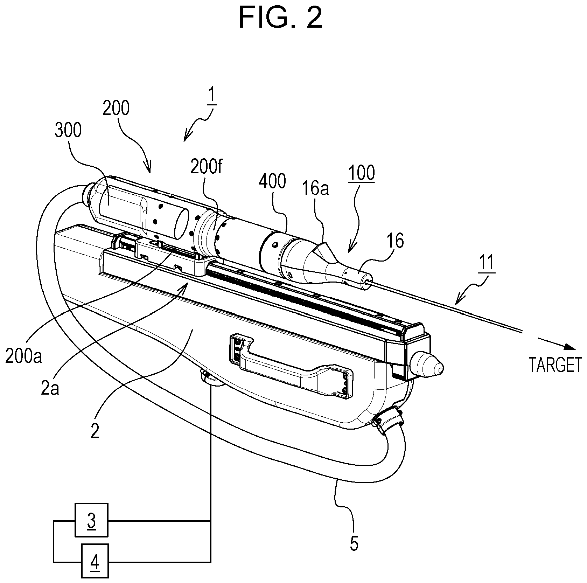

A medical system 1A and a medical device 1 including a continuum robot will be described with reference to Figs. 1 and 2. Fig. 1 is an overall view of the medical system 1A. Fig. 2 is a perspective view illustrating the medical device 1 and a support base 2.

The medical system 1A is an example of a continuum robot system and includes the medical device 1, the support base 2 supporting the medical device 1, and a control apparatus 3 that controls the medical device 1. In the present Example, the medical system 1A includes a monitor 4 as a display device.

The medical device 1 includes a catheter unit (bendable unit) 100 having a catheter 11 as a bendable body, and a base unit (drive unit, unit for attachment) 200. The catheter unit 100 is detachably attached to the base unit 200.

In the present Example, a user of the medical system 1A and the medical device 1 inserts the catheter 11 into a target, and performs work such as observing the inside of the target, collecting various specimens from the inside of the target, and treating the inside of the target. As one embodiment, the user can insert the catheter 11 into a patient as the target. Specifically, the user can perform work such as observing, collecting, and resecting a lung tissue by inserting the catheter 11 into a bronchus through an oral cavity or a nasal cavity.

The catheter 11 can be used as a guide (sheath) that guides a medical tool for performing the above-described work. Examples of the medical tool (tool) are an endoscope, forceps, an abrasion device, and the like. In addition, the catheter 11 itself may have a function as the above-described medical tool.

In the present Example, the control apparatus 3 is an example of control means according to the present invention, and includes a computing device 3a and an input device 3b. The input device 3b receives, from the user, a command and input for operating the catheter 11 and the support base 2. The computing device 3a includes a storage storing a program for controlling the catheter 11 and the support base 2 and various kinds of data, a random-access memory, and a central processing unit for executing the program. In addition, the control apparatus 3 may include an output unit that outputs a signal for displaying an image on the monitor 4.

As illustrated in Fig. 2, in the present Example, the medical device 1 is electrically connected to the control apparatus 3 via the support base 2 and a cable 5 coupling the base unit 200 of the medical device 1 to the support base 2. The medical device 1 may be directly connected to the control apparatus 3 via a cable. The medical device 1 may be wirelessly connected to the control apparatus 3. Further, in Figs. 1 and 2, the control apparatus 3 is illustrated as an independent constituent component, but may be included in the base unit 200 or the support base 2.

The medical device 1 is detachably attached to the support base 2 via the base unit 200. More specifically, in the medical device 1, an attachment part (connection part) 200a of the base unit 200 is detachably attached to a moving stage (receiving part) 2a of the support stage 2. Even in a state in which the attachment part 200a of the medical device 1 is detached from the moving stage 2a, the connection of the medical device 1 and the control apparatus 3 is maintained so that the medical device 1 can be controlled by the control apparatus 3. In the present Example, even in a state in which the attachment part 200a of the medical device 1 is detached from the moving stage 2a, the medical device 1 and the support base 2 are connected to each other via the cable 5.

The user can manually move the medical device 1 and insert the catheter 11 into the target in a state in which the medical device 1 is detached from the support base 2 (a state in which the medical device 1 is detached from the moving stage 2a).

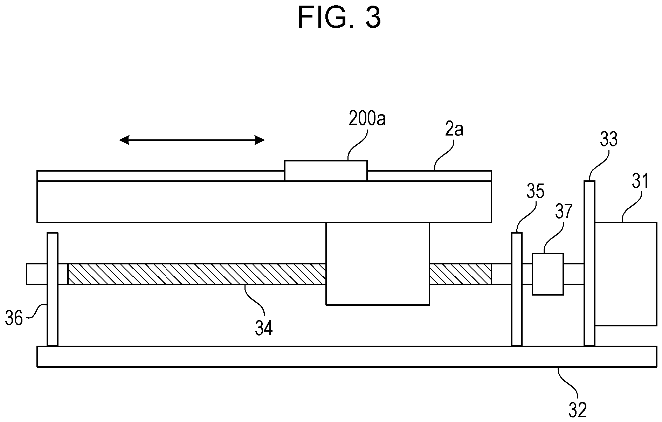

In addition, in a state in which the catheter 11 is inserted in the target and the medical device 1 is attached to the support base 2, the user can use the medical device 1. Specifically, the medical device 1 is moved as the moving stage 2a is moved in a state in which the medical device 1 is attached to the moving stage 2a. Then, an operation of moving the moving stage 2a in a direction in which the catheter 11 is inserted into the target, and an operation of moving the moving stage 2a in a direction in which the catheter 11 is pulled out of the target are performed. The movement of the moving stage 2a is performed by the control apparatus 3 controlling a moving motor 31 (see Fig. 3) disposed in the support base 2.

The medical device 1 includes a wire drive section (linear member drive section, line drive section, main body drive section) 300 for driving the catheter 11. In the present Example, the medical device 1 is a robot catheter device that drives the catheter 11 by the wire drive section 300 controlled by the control apparatus 3.

The control apparatus 3 can control the wire drive section 300 to perform an operation of flexing the catheter 11. In the present Example, the wire drive section 300 is included in the base unit 200. More specifically, the base unit 200 includes a base housing 200f housing the wire drive section 300. That is, the base unit 200 includes the wire drive section 300.

In this case, an end at which a tip of the catheter 11 that is to be inserted into the target is disposed in an extension direction of the catheter 11 is referred to as a distal end. An end opposite to the distal end in the extension direction of the catheter 11 is referred to as a proximal end.

The catheter unit 100 includes a proximal end cover 16 covering the proximal end side of the catheter 11. The proximal end cover 16 has a tool hole 16a. The medical tool can be inserted into the catheter 11 through the tool hole 16a.

As described above, in the present Example, the catheter 11 has a function as a guide device for guiding the medical tool to a desired position within the target.

For example, in a state in which an endoscope is inserted in the catheter 11, the catheter 11 is inserted to a target position within the target. In this case, at least one of a manual operation by the user, the movement of the moving stage 2a, and the driving of the catheter 11 by the wire drive section 300 is used. After the catheter 11 reaches the target position, the endoscope is pulled out of the catheter 11 through the tool hole 16a. Then, work such as inserting the medical tool from the tool hole 16a and collecting various specimens from the inside of the target and treating the inside of the target is performed.

As described above, the catheter unit 100 is configured to be detachably attached to the base unit 200. After the medical device 1 is used, the user detaches the catheter unit 100 from the base unit 200, attaches a new catheter unit 100 to the base unit 200, and can use the medical device 1 again.

As illustrated in Fig. 2, the medical device 1 includes an operation section 400. In the present Example, the operation section 400 is included in the catheter unit 100 and configured to be rotatable. The operation section 400 is operated by the user to fix the catheter unit 100 to the base unit 200 for attachment of the catheter unit 100 to the base unit 200 and to release the fixation of the catheter unit 100 to the base unit 200 for detachment of the catheter unit 100 from the base unit 200.

In the medical system 1A configured as described above, for example, an image captured by the endoscope can be displayed on the monitor 4 by connecting the endoscope inserted in the catheter 11 to the monitor 4. In addition, information regarding the state of the medical device 1 and the control of the medical device 1 can be displayed on the monitor 4 by connecting the monitor 4 to the control apparatus 3. For example, information regarding the position of the catheter 11 in the target and navigation of the catheter 11 in the target can be displayed on the monitor 4. The monitor 4 may be connected to the control apparatus 3 and the endoscope by wire or wirelessly. Further, the monitor 4 and the control apparatus 3 may be connected to each other via the support base 2.

### <Support Base>

The support base 2 will be described with reference to Fig. 3. Fig. 3 is an explanatory diagram of the support base 2.

The support base 2 includes a stay 32. The moving motor 31 including a stepping motor is fixed to a motor holder 33 attached to the stay 32. An end of a lead screw 34 is connected to a shaft of the moving motor 31 via a coupling 37. The lead screw 34 is rotatably supported by lead screw stays 35 and 36 including bearings not illustrated. The moving stage 2a to which the medical device 1 is fixed via the attachment part 200a is connected to the lead screw 34. The lead screw 34 is a so-called feed screw mechanism, and enables the moving stage 2a to slide in an arrow direction illustrated in the drawing by converting a rotational motion of the moving motor 31 into a linear motion. As described above, the moving motor 31 is a drive source that slides the moving stage 2a. The control apparatus 3 drives the moving motor 31 and controls movement of the moving stage 2a.

### <Catheter>

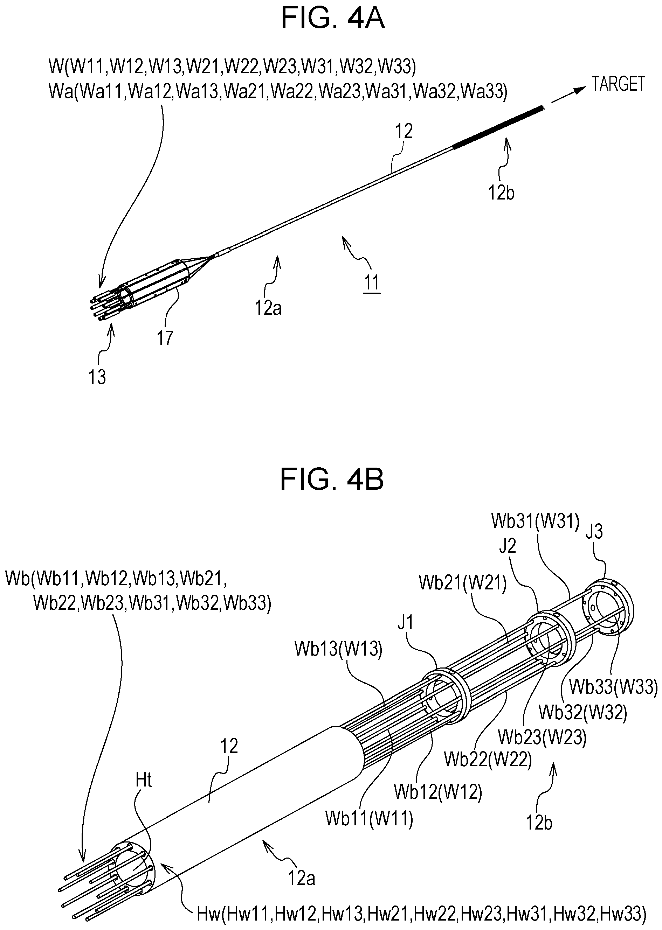

The catheter 11 as the bendable body will be described with reference to Figs. 4a and 4B. Figs. 4A and 4B are explanatory diagrams of the catheter 11. Fig. 4A is a diagram explaining the entire catheter 11. Fig. 4B is an enlarged diagram of the catheter 11.

The catheter 11 includes a bending part (bending body, catheter body) 12 and a bending drive section (catheter drive section) 13 configured to bend the bending part 12. The bending drive section 13 is configured to receive a drive force of the wire drive section 300 through a coupling device 21 described later and bend the bending part 12.

The catheter 11 is extended in the direction in which the catheter 11 is inserted into the target. The extension direction (longitudinal direction) of the catheter 11 is the same as an extension direction (longitudinal direction) of the bending part 12 and extension directions (longitudinal directions) of first to ninth drive wires (W11 to W33) described later.

The bending drive section 13 includes a plurality of drive wires (drive lines, linear members, linear actuators) connected to the bending part 12. Specifically, the bending drive section 13 includes the first drive wire W11, the second drive wire W12, the third drive wire W13, the fourth drive wire W21, the fifth drive wire W22, the sixth drive wire W23, the seventh drive wire W31, the eighth drive wire W32, and the ninth drive wire W33. The first to ninth drive wires (W11 to W33) are arranged side by side along a virtual circle having a predetermined radius.

Each of the first to ninth drive wires (W11 to W33) includes a part (shaft to be held, rod) Wa to be held. Specifically, the first drive wire W11 includes the first part Wa11 to be held. The second drive wire W12 includes the second part Wa12 to be held. The third drive wire W13 includes the third part Wal3 to be held. The fourth drive wire W21 includes the fourth part Wa21 to be held. The fifth drive wire W22 includes the fifth part Wa22 to be held. The sixth drive wire W23 includes the sixth part Wa23 to be held. The seventh drive wire W31 includes the seventh part Wa31 to be held. The eighth drive wire W32 includes the eighth part Wa32 to be held. The ninth drive wire W33 includes the ninth part Wa33 to be held.

In the present Example, the first to ninth parts (Wall to Wa33) to be held have the same shape.

Each of the first to ninth drive wires (W11 to W33) has a flexible wire body (line body, linear body) Wb. Specifically, the first drive wire W11 includes the first wire body Wb11. The second drive wire W12 includes the second wire body Wb12. The third drive wire W13 includes the third wire body Wb13. The fourth drive wire W21 includes the fourth wire body Wb21. The fifth drive wire W22 includes the fifth wire body Wb22. The sixth drive wire W23 includes the sixth wire body Wb23. The seventh drive wire W31 includes the seventh wire body Wb31. The eighth drive wire W32 includes the eighth wire body Wb32. The ninth drive wire W33 includes the ninth wire body Wb33.

In the present Example, the first to third wire bodies (Wb11 to Wb13) have the same shape. The fourth to sixth bodies (Wb21 to Wb23) have the same shape. The seventh to ninth bodies (Wb31 to Wb33) have the same shape. In the present Example, the first to ninth wire bodies (Wb11 to Wb33) have the same shape, except for the lengths.

The first to ninth parts (Wall to Wa33) to be held are fixed to the first to ninth wire bodies (Wb11 to Wb33) at proximal ends of the first to ninth wire bodies (Wb11 to Wb33) .

The first to ninth drive wires (W11 to W33) are inserted into the bending part 12 through a wire guide 17 and fixed to the bending part 12 via the wire guide 17.

Any one of the first to ninth drive wires (W11 to W33) can be referred to as a drive wire W. In the present Example, the first to ninth drive wires (W11 to W33) have the same shape, except for the lengths of the first to ninth wire bodies (Wb11 to Wb33).

In the present Example, the bending part 12 is a tube member that is flexible and has a path Ht for insertion of the medical tool.

A plurality of wire holes are provided in a wall surface of the bending part 12 for the first to ninth drive wires (W11 to W33) to pass through the wire holes. Specifically, the first wire hole Hw11, the second wire hole Hw12, the third wire hole Hw13, the fourth wire hole Hw21, the fifth wire hole Hw22, the sixth wire hole Hw23, the seventh wire hole Hw31, the eighth wire hole Hw32, and the ninth wire hole Hw33 are provided in the wall surface of the bending part 12. The first to ninth wire holes Hw (Hw11 to Hw33) correspond to the first to ninth drive wires (W11 to W33), respectively. The numbers after the reference sign Hw indicate the numbers of the corresponding drive wires. For example, the first drive wire W11 is inserted into the first wire hole Hw11.

Any one of the first to ninth wire holes (Hw11 to Hw33) can be referred to as a wire hole Hw. In the present Example, the first to ninth wire holes (Hw11 to Hw33) have the same shape.

The bending part 12 includes an intermediate region 12a and a bending region 12b. The bending region 12b is disposed at a distal end of the bending part 12. In the bending region 12b, a first guide ring J1, a second guide ring J2, and a third guide ring J3 are disposed. The bending region 12b is a region in which the magnitude and direction of flexing of the bending part 12 can be controlled by the bending drive section 13 moving the first guide ring J1, the second guide ring J2, and the third guide ring J3. In Fig. 4B, an illustration of a portion of the bending part 12 that covers the first to third guide rings (J1 to J3) is omitted.

The medical tool is guided to the tip of the catheter 11 by the path Ht and the first to third guide rings (J1 to J3) .

Each of the first to ninth drive wires (W11 to W33) extends through the intermediate region 12a and is fixed to each of the first to third guide rings (J1 to J3).

Specifically, the first drive wire W11, the second drive wire W12, and the third drive wire W13 are fixed to the first guide ring J1. The fourth drive wire W21, the fifth drive wire W22, and the sixth drive wire W23 penetrate through the first guide ring J1 and are fixed to the second guide ring J2. The seventh drive wire W31, the eighth drive wire W32, and the ninth drive wire W33 penetrate through the first guide ring J1 and the second guide ring J2 and are fixed to the third guide ring J3.

The medical device 1 can bend the bending part 12 toward a direction intersecting the extension direction of the catheter 11 by causing the wire drive section 300 to drive the bending drive section 13. Specifically, the medical device 1 can bend the bending region 12b of the bending part 12 in the direction intersecting the extension direction of the bending part 12 via the first to the third guide rings (J1 to J3) by moving each of the first to ninth drive wires (W11 to W33) in the extension direction of the bending part 12.

The user can insert the catheter 11 to a target part within the target by using any one of the movement of the medical device 1 by hand or the moving stage 2a and bending of the bending part 12.

### <Base Unit>

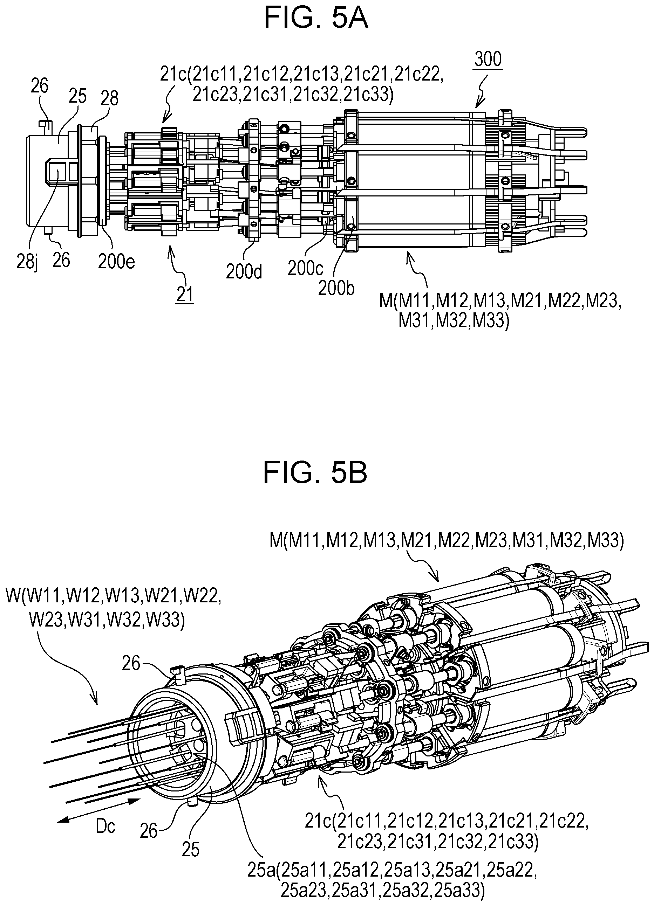

The base unit 200 and the wire drive section 300 will be described with reference to Fig. 5A and Fig. 5B.

Figs. 5A and 5B are explanatory diagrams of the base unit 200 and the wire drive section 300. Fig. 5A is a side view illustrating an inner structure of the base unit 200. Fig. 5B is a perspective view illustrating coupling of the wire drive section 300, the coupling device 21, and the bending drive section 13.

The wire drive section 300 includes a plurality of drive sources (motors). In the present Example, the wire drive section 300 includes a first drive source M11, a second drive source M12, a third drive source M13, a fourth drive source M21, a fifth drive source M22, a sixth drive source M23, a seventh drive source M31, an eighth drive source M32, and a ninth drive source M33.

Any one of the first to ninth drive sources (M11 to M33) can be referred to as a drive source M. In the present Example, the first to ninth drive sources (M11 to M33) have the same configuration.

The base unit 200 includes the coupling device 21. The coupling device 21 is connected to the wire drive section 300. The coupling device 21 includes a plurality of coupling parts. In the present Example, the coupling device 21 includes a first coupling part 21c11, a second coupling part 21c12, a third coupling part 21c13, a fourth coupling part 21c21, a fifth coupling part 21c22, a sixth coupling part 21c23, a seventh coupling part 21c31, an eighth coupling part 21c32, and a ninth coupling part 21c33.

Any one of the first to ninth coupling parts (21c11 to 21c33) can be referred to as a coupling part 21c. In the present Example, the first to ninth coupling parts (21c11 to 21c33) have the same configuration.

Each of the plurality of coupling parts is connected to a respective one of the plurality of drive sources and driven by a respective one of the plurality of drive sources. Specifically, the first coupling part 21c11 is connected to the first drive source M11 and driven by the first drive source M11. The second coupling part 21c12 is connected to the second drive source M12 and driven by the second drive source M12. The third coupling part 21c13 is connected to the third drive source M13 and driven by the third drive source M13. The fourth coupling part 21c21 is connected to the fourth drive source M21 and driven by the fourth drive source M21. The fifth coupling part 21c22 is connected to the fifth drive source M22 and driven by the fifth drive source M22. The sixth coupling part 21c23 is connected to the sixth drive source M23 and driven by the sixth drive source M23. The seventh coupling part 21c31 is connected to the seventh drive source M31 and driven by the seventh drive source M31. The eighth coupling part 21c32 is connected to the eighth drive source M32 and driven by the eighth drive source M32. The ninth coupling part 21c33 is connected to the ninth drive source M33 and driven by the ninth drive source M33.

As illustrated in Fig. 5B, the bending drive section 13 including the first to ninth drive wires (W11 to W33) of the catheter 11 is coupled to the coupling device 21. The bending drive section 13 receives a drive force of the wire drive section 300 through the coupling device 21 and bends the bending drive section 12.

The drive wire W is coupled to the coupling part 21c via the part Wa to be held. Each of the plurality of drive wires is coupled to a respective one of the plurality of coupling parts.

Specifically, the first part Wa11 that is to be held and is included in the first drive wire W11 is coupled to the first coupling part 21c11. The second part Wa12 that is to be held and is included in the second drive wire W12 is coupled to the second coupling part 21c12. The third part Wa13 that is to be held and is included in the third drive wire W13 is coupled to the third coupling part 21c13. The fourth part Wa21 that is to be held and is included in the fourth drive wire W21 is coupled to the fourth coupling part 21c21. The fifth part Wa22 that is to be held and is included in the fifth drive wire W22 is coupled to the fifth coupling part 21c22. The sixth part Wa23 that is to be held and is included in the sixth drive wire W23 is coupled to the sixth coupling part 21c23. The seventh part Wa31 that is to be held and is included in the seventh drive wire W31 is coupled to the seventh coupling part 21c31. The eighth part Wa32 that is to be held and is included in the eighth drive wire W32 is coupled to the eighth coupling part 21c32. The ninth part Wa33 that is to be held and is included in the ninth drive wire W33 is coupled to the ninth coupling part 21c33.

The base unit 200 includes a base frame 25. The base frame 25 has a plurality of insertion holes through which the first to ninth drive wires (W11 to W33) extend. The base frame 25 has the first insertion hole 25a11, the second insertion hole 25a12, the third insertion hole 25a13, the fourth insertion hole 25a21, the fifth insertion hole 25a22, the sixth insertion hole 25a23, the seventh insertion hole 25a31, the eighth insertion hole 25a32, and the ninth insertion hole 25a33. The first to ninth insertion holes (25a11 to 25a33) correspond to the first to ninth drive wires (W11 to W33), respectively. The numbers after the reference sign 25a indicate the numbers of the corresponding drive wires. For example, the first drive wire W11 is inserted into the first insertion hole 25a11.

Any one of the first to ninth insertion holes (25a11 to 25a33) can be referred to as an insertion hole 25a. In the present Example, the first to ninth insertion holes (25a11 to 25a33) have the same shape.

The first to ninth drive wires (W11 to W33) engage with the corresponding first to ninth insertion holes (25a11 to 25a33) and the corresponding first to ninth coupling parts (21c11 to 21c33), respectively.

The base frame 25 includes a key receiving part (key hole, base side key, main body side key) 22 that receives a key shaft included in the catheter unit 100. The catheter unit 100 is attached to the base unit 200 in a correct phase since the key shaft 15 engages with the key receiving part 22.

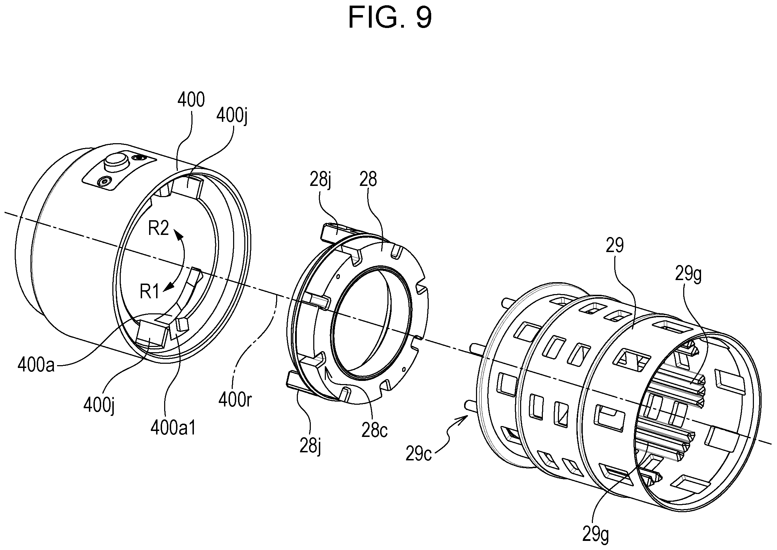

In addition, the base frame 25 includes a connection shaft 26. As illustrated in Fig. 9, a connection groove 400a that engages with the connection shaft 26 is disposed on the inner side of the operation section 400. When the catheter unit 100 is attached to the base unit 200, the connection shaft 26 engages with the connection groove 400a via an entrance port 400a1 and moves along the connection groove 400a according to the rotational position (see a locking direction R1 and an unlocking direction R2) of the operation section 400.

### <Coupling of Driving Sources to Drive Wires>

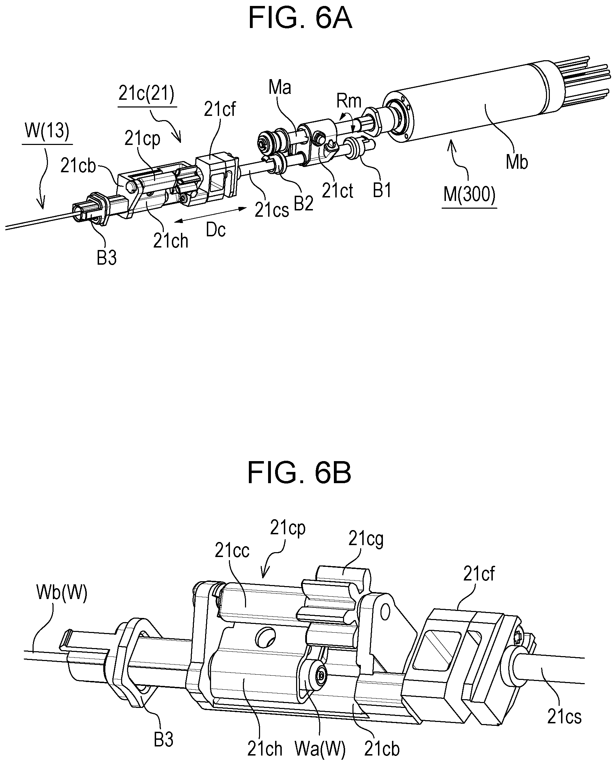

The coupling of the wire drive section 300, the coupling device 21, and the bending drive section 13 will be described with reference to Figs. 6A and 6B. Figs. 6A and 6B are explanatory diagrams of the wire drive section 300, the coupling device 21, and the bending drive section 13. Fig. 6A is a perspective view of the drive source M, the coupling part 21c, and the drive wire W. Fig. 6B is an enlarged view of the coupling part 21c and the drive wire W.

In the present Example, configurations in which the first to ninth drive wires (W11 to W33) are coupled to the first to ninth coupling parts (21c11 to 21c33), respectively, are the same. In addition, configurations in which the first to ninth coupling parts (21c11 to 21c33) are connected to the first to ninth drive sources (M11 to M33), respectively, are the same. Therefore, in the following description, a single drive wire W, a single coupling part 21c, and a single drive source M are used to explain a configuration in which the single drive wire W, the single coupling part 21c, and the single drive source M are connected to each other.

As illustrated in Fig. 6A, the drive source M includes an output shaft Ma that is a motor shaft, and a motor body Mb that rotates the output shaft Ma in a rotation direction Rm. A spiral groove is disposed on a surface of the output shaft Ma. The output shaft Ma has a so-called screw shape. The motor body Mb is fixed to a motor flame 200b.

The coupling part 21c includes a tractor 21ct connected to the output shaft Ma, and a tractor support shaft 21cs supporting the tractor 21ct. The tractor support shaft 21cs is connected to a coupling base 21cb via a force sensor 21cf. The force sensor 21cf functions as a tensile force detector and outputs, to the control apparatus 3, an electric signal that is a detected value according to a tensile force that has occurred in the drive wire W. The force sensor 21cf will be described later in detail.

The coupling part 21c includes a plate spring 21ch as a holding part for holding the part Wa that is to be held and is included in the drive wire W. The drive wire W extends through the insertion hole 25a and engages with the coupling part 21c. More specifically, the part Wa to be held engages with the plate spring 21ch. As described later, the plate screw 21ch can be in a state (fixing state) in which the plate screw 21ch sandwiches and fixes the part Wa to be held and a state (releasing state) in which the plate screw 21ch releases the part Wa to be held.

The coupling part 21c includes a pressing member 21cp. The pressing member 21cp includes a gear part 21cg engaging with an internal gear 29 described later, and a cam 21cc as a pressing part for pressing the plate spring 21ch.

As described later, the cam 21cc can move with respect to the plate spring 21ch. As the cam 21cc moves, the state of the plate spring 21ch is switched between the fixing state and the releasing state.

As illustrated in Fig. 6A, the coupling part 21c is supported by a first bearing B1, a second bearing B2, and a third bearing B3. The first bearing B1 is supported by a first bearing frame 200c of the base unit 200. The second bearing B2 is supported by a second bearing frame 200d of the base unit 200. The third bearing B3 is supported by a third bearing frame 200e of the base unit 200.

Therefore, when the output shaft Ma rotates in the rotation direction Rm, the rotation of the coupling part 21c around the output shaft Ma is restricted. The first bearing B1, the second bearing B2, and the third bearing B3 are provided for the first to ninth coupling parts (21c11 to 21c33) .

Since the rotation of the coupling part 21c around the output shaft Ma is restricted, when the output shaft Ma rotates, a force along the rotational axis direction of the output shaft Ma acts on the tractor 21ct due to the spiral groove of the output shaft Ma. As a result, the coupling part 21c moves along the rotational axis direction of the output shaft Ma (Dc direction). As the coupling part 21c moves, the drive wire W moves and the bending part 12 bends. That is, the output shaft Ma and the tractor 21ct forms a so-called feed screw that converts a rotational motion transmitted from the drive source M into a linear motion by the screw.

The control apparatus 3 independently controls each of the first to ninth drive sources (M11 to M33). That is, any drive source among the first to ninth drive sources (M11 to M33) can independently operate or stop regardless of whether or not the other drive sources are in a stopped state. In other words, the control apparatus 3 can independently control each of the first to ninth drive wires (W11 to W33). As a result, each of the first to third guide rings (J1 to J3) can be independently controlled and the bending region 12b of the bending part 12 can be flexed in any direction.

Specifically, the third guide ring J3 is disposed at the proximal end of the bending part 12. The seventh to ninth drive wires (W31 to W33) are connected to the third guide ring J3, and the seventh to ninth drive sources (M31 to M33 are connected to the three drive wires. That is, by controlling the seventh to ninth drive sources (M31 to M33), the orientation of the third guide ring J3 can be changed to any direction, and the bending region 12b bends into an arc along the third guide ring J3. Similarly, by controlling the fourth to sixth drive sources (M21 to M23), the orientation of the second guide ring J2 can be changed to any direction via the fourth to sixth drive wires (W21 to W23). Similarly, by controlling the first to third drive sources (M11 to M13), the orientation of the first guide ring J1 can be changed to any direction via the first to third drive wires (W11 to W13).

As described above, since each of the three guide rings (J1 to J3) can be independently oriented in any direction, the bending region 12b can be in a complex shape. Therefore, the catheter 11 can be inserted into and moved in a long and thin organ having a complex shape, such as the bronchus and a digestive organ inside the patient's body.

### <Force Sensor>

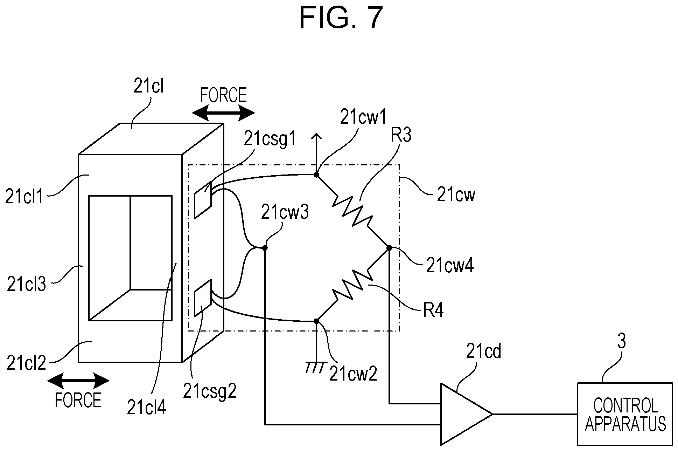

The force sensor 21cf will be described with reference to Fig. 7. Fig. 7 is an explanatory diagram of the force sensor 21cf.

As illustrated in Figs. 6A and 6B, the force sensor 21cf that detects a tensile force that has occurred in the drive wire W is disposed in the coupling part 21c of the base unit 200. Since this tensile force occurs when the catheter 11 comes into contact with the inner wall of the organ, it is possible to determine whether or not the catheter 11 is smoothly moving in the organ.

As illustrated in Fig. 7, the force sensor 21cf includes a strain-generating body 21cl and a strain gauge 21csg attached to the strain-generating body 21cl, and the strain gauge 21csg detects a slight elastic deformation of the strain-generating body 21cl caused by the occurrence of a tensile force in the drive wire W.

The strain-generating body 21cl is a square-shaped metal component with an opening in the middle of the strain-generating body 21cl. The tensile force that has occurred in the drive wire W causes upper and lower horizontal walls 21cl1 and 21cl2 of the strain-generating body 21cl to move in arrow directions in the drawing. For example, when the horizontal wall 21cl1 moves rightward in the drawing, the horizontal wall 21cl2 moves leftward. Vertical walls 21cl3 and 21cl4 of the strain-generating body 21cl slightly elastically deform due to positional shifts of the horizontal walls 21cl1 and 21cl2. The elastic deformations are detected by the strain gauge 21csg attached to the vertical walls 21cl3 and 21cl4. In the Example, two strain gauges 21csg1 and 21csg2 are attached. The strain gauge 21csg deforms according to the expansion and contraction of an attachment target object and measures an amount of strain of the target object by changing a resistance value of the strain gauge 21csg.

These strain gauges 21csg1 and 21csg2 and resistors R3 and R4 form a wheatstone bridge circuit 21cw including the strain gauges 21csg1 and 21csg2, and the wheatstone bridge circuit 21cw is configured to convert a small change in a resistance value of the strain gauge 21csg into a voltage. A voltage is applied between input terminals 21cw1 and 21cw2 of the wheatstone bridge circuit 21cw, and a differential amplifier 21cd amplifies the difference in electric potential between output terminals 21cw3 and 21cw4 and outputs the output voltage to the control apparatus 3. The difference in electric potential between the output terminals 21cw3 and 21cw4 corresponds to the tensile force that has occurred in the drive wire W, and thus the control apparatus 3 can detect, based on the output voltage of the differential amplifier 21ce, the tensile force that has occurred in the drive wire W. Although the wheatstone bridge circuit 21cw includes the two strain gauges 21csg1 and 21csg2, one or more of four resistors forming the wheatstone bridge circuit 21cw may be a strain gauge.

### <Fixation and Release of Fixation of Bending Drive Section>

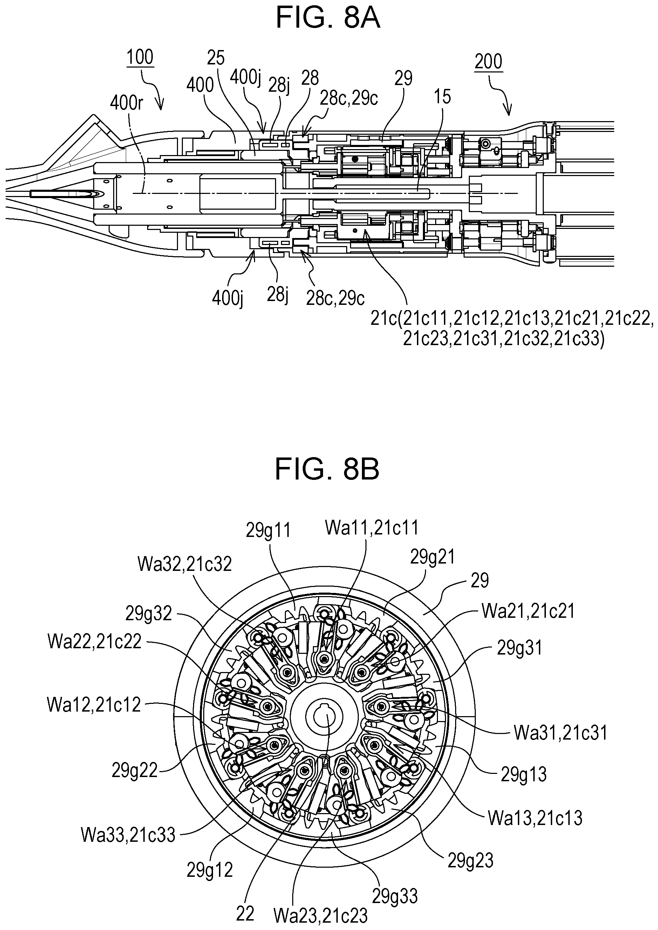

A configuration for fixing the bending drive section 13 to the coupling device 21 and a configuration for releasing the fixation of the bending drive section 13 by the coupling device 21 will be described with reference to Figs. 8A, 8B, 9, 10, 11, 12, 13, and 14.

Figs. 8A and 8B are diagrams explaining the coupling of the catheter unit 100 and the base unit 200. Fig. 8A is a cross-sectional view of the catheter unit 100 and the base unit 200. Fig. 8A is a cross-sectional view of the catheter unit 100 and the base unit 200 taken along a rotational axis 400r. Fig. 8B is a cross-sectional view of the base unit 200. Fig. 8B is a cross-sectional view of a portion of the coupling part 21c of the base unit 200 taken along a direction orthogonal to the rotational axis 400r.

Fig. 9 is an exploded view explaining the coupling of the catheter unit 100 and the base unit 200.

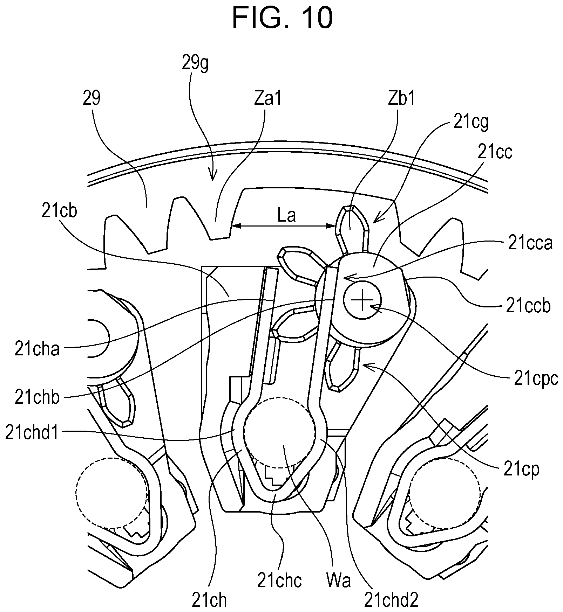

Figs. 10, 11, 12, 13, and 14 are diagrams explaining the fixation of the drive wire W by the coupling part 21c.

As illustrated in Figs. 8A and 9, the base unit 200 includes a joint (intermediate member, second transmission member) 28 and the internal gear 29 as a moving gear (interlocking gear, transmission member, first transmission member) that interlocks with the operation section 400 via the joint 28.

The joint 28 includes a plurality of transmission parts 28c. The internal gear 29 includes a plurality of transmission receiving parts 29c. The plurality of transmission parts 28c engage with the plurality of transmission parts 29c. When the joint 28 rotates, the rotation of the joint 28 is transmitted to the internal gear 29.

When the catheter unit 100 is attached to the base unit 200, an engagement part 400j included in the operation section 400 engages with a joint engagement part 28j of the joint 28. When the operation section 400 rotates, the rotation of the operation section 400 is transmitted to the joint 28. The operation section 400, the joint 28, and the internal gear 29 rotate in the same direction.

The internal gear 29 includes a plurality of tooth parts for switching between a state in which the first to ninth coupling parts (21c11 to 21c33) fix the first to ninth drive wires (W11 to W33), respectively, and a state in which the first to ninth coupling parts (21c11 to 21c33) release the first to ninth drive wires (W11 to W33), respectively. Each of the plurality of tooth parts (acting parts, switching gear parts) of the internal gear 29 engages with a respective one of the gear parts 21cg of the pressing members 21cp included in the first to ninth coupling parts (21c11 to 21c33).

Specifically, in the present Example, the internal gear 29 includes a first tooth part 29g11, a second tooth part 29g12, a third tooth part 29g13, a fourth tooth part 29g21, a fifth tooth part 29g22, a sixth tooth part 29g23, a seventh tooth part 29g31, an eighth tooth part 29g32, and a ninth tooth part 29g33. Each of the first to ninth tooth parts (29g11 to 29g33) is formed with a gap between the tooth parts.

The first tooth part 29g11 engages with the gear part 21cg of the first coupling part 21c11. The second tooth part 29g12 engages with the gear part 21cg of the second coupling part 21c12. The third tooth part 29g13 engages with the gear part 21cg of the third coupling part 21c13. The fourth tooth part 29g21 engages with the gear part 21cg of the fourth coupling part 21c21. The fifth tooth part 29g22 engages with the gear part 21cg of the fifth coupling part 21c22. The sixth tooth part 29g23 engages with the gear part 21cg of the sixth coupling part 21c23. The seventh tooth part 29g31 engages with the gear part 21cg of the seventh coupling part 21c31. The eighth tooth part 29g32 engages with the gear part 21cg of the eighth coupling part 21c32. The ninth tooth part 29g33 engages with the gear part 21cg of the ninth coupling part 21c33.

Any one of the first to ninth tooth parts (29g11 to 29g33) can be referred to as a tooth part 29g. In the present Example, the first to ninth tooth parts (29g11 to 29g33) have the same configuration.

In the present Example, configurations in which the first to ninth coupling parts (21c11 to 21c33) are coupled to the first to ninth drive wires (W11 to W33), respectively, are the same. In addition, configurations in which the first to ninth tooth parts (29g11 to 29g33) are connected to the first to ninth coupling parts (21c11 to 21c33), respectively, are the same. Therefore, in the following description, a single drive wire W, a single coupling part 21c, and a single tooth part 29g are used to explain a configuration in which the single drive wire W, the single coupling part 21c, and the single tooth part 29g are connected to each other.

In each of the first to ninth coupling parts (21c11 to 21c33), as the gear part 21cg is moved by the internal gear 29, the pressing member 21cp rotates and the cam 21cc moves to a pressing position and a retracted position where the cam 21cc is retracted from the pressing position.

The internal gear 29 rotates as the operation section 400 is rotated. Each of the first to ninth coupling parts (21c11 to 21c33) operates as the internal gear 29 rotates. As described above, the first to ninth coupling parts (21c11 to 21c33) can be operated by the operation of rotating the single operation section 400.

In a state in which the catheter unit 100 is attached to the base unit 200, the operation section 400 can move to a fixation position and a detachment position. In addition, as described later, in a state in which the catheter unit 100 is attached to the base unit 200, the operation section 400 can move to a release position. The release position is located between the fixation position and the detachment position in a rotation direction of the operation section 400. In a state in which the operation section 400 is present at the detachment position, the catheter unit 100 is attached to the base unit 200 (temporarily attached state).

In the temporarily attached state, the drive wire W is not fixed (locked) to the coupling part 21c. This state is referred to as an unlocking state of the coupling part 21c. A state in which the drive wire W is fixed (locked) to the coupling part 21c is referred to as a locking state of the coupling part 21c.

An operation of fixing the drive wire W to the coupling part 21c will be described with reference to Figs. 10, 11, 12, 13, and 14.

After the temporarily attached state, that is, after the catheter unit 100 is attached to the base unit 200, and before the operation section 400 is operated, the catheter unit 100 is enabled to be detached from the base unit 200. A state in which the catheter unit 100 is enabled to be detached from the base unit 200 is referred to as a detachment-enabled state.

Fig. 10 is a diagram illustrating states of the internal gear 29 and the coupling part 21c in the detachment-enabled state. The plate spring 21ch of the coupling part 21c includes a part 21cha fixed to the coupling base 21cb, and a part 21chb that is to be pressed and comes into contact with the cam 21cc of the pressing member 21cp. The plate spring 21ch includes a first portion 21chd1 and a second portion 21chd2. When the catheter unit 100 is attached to the base unit 200, the part Wa to be held is inserted into a gap between the first portion 21chd1 and the second portion chd2.

The cam 21cc includes a holding surface 21cca and a pressing surface 21ccb. The holding surface 21cca is closer to the center 21cpc of rotation of the pressing member 21cp than the pressing surface 21ccb in a rotation radius direction of the pressing member 21cp.

As illustrated in Fig. 10, in the detachment-enabled state (state in which the operation section 400 is present at the detachment position), the plate spring 21ch is held at a position where the part 21chb to be pressed is in contact with the holding surface 21cca. In addition, a tooth Za1 of the internal gear 29 and a tooth Zb1 of the gear part 21cg are stopped in a state in which a clearance La occurs between the tooth Za1 and the tooth Zb1.

A direction in which the operation section 400 moves from the detachment position to the release position and the fixation position in the rotation direction of the operation section 400 is referred to as a locking direction (fixation direction). A direction in which the operation section 400 moves from the fixation position to the release position and the detachment position in the rotation direction of the operation section 400 is referred to as an unlocking direction. The operation section 400 rotates from the release position in the unlocking direction and moves to the detachment position. The operation section 400 rotates from the release position in the locking direction and moves to the fixation position.

In the temporarily attached state, the coupling part 21c is in the unlocking state, and the fixation of the drive wire W by the coupling part 21c is released.

When the coupling part 21c is in the unlocking state, the cam 21cc is located at the retracted position where the cam 21cc is retracted from the pressing position described later. In this case, the fixation of the part Wa, which is to be held, by the plate spring 21ch is released. A force with which the first portion 21chd1 and the second portion 21chd2 tighten the part Wa to be held when the coupling part 21c is in the unlocking state is less than a force with which the first portion 21chd1 and the second portion 21chd2 tighten the part Wa to be held when the coupling part 21c is in the locking state.

In a case where the catheter unit is moved in a detachment direction Dd with respect to the base unit 200 in a state in which the coupling part 21c is in the unlocking state, the part Wa to be held can be pulled out of the gap between the first portion 21chd1 and the second portion 21chd2.

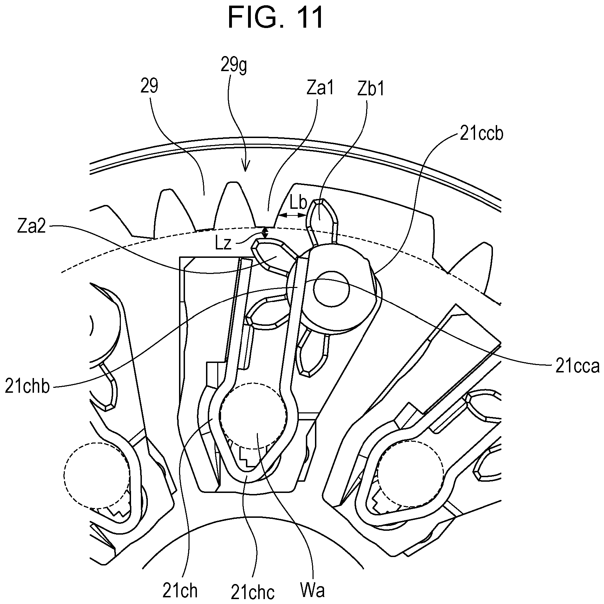

Fig. 11 is a diagram illustrating states of the internal gear 29 and the coupling part 21c when the operation section 400 is rotated from the detachment position in the locking direction. Fig. 11 is a diagram illustrating states of the internal gear 29 and the coupling part 21c in a state in which the operation section 400 is present at the release position.

When the operation section 400 is rotated in the locking direction in a state (Fig. 10) in which the operation section 400 is present at the detachment position, the internal gear 29 rotates clockwise. Then, the operation section 400 is located at the release position.

Even when the operation section 400 is rotated, the key shaft 15 engages with the key receiving part 22, and thus the rotation of the entire catheter unit 100 (excluding the operation section 400) with respect to the base unit 200 is restricted. That is, the operation section 400 can rotate with respect to the catheter unit 100 and the base unit 200 in a state in which the entire catheter unit 100 (excluding the operation section 400) and the base unit 200 are stopped.

As the internal gear 29 rotates clockwise, the clearance between the tooth Za1 of the internal gear 29 and the tooth Zb1 of the gear part 21cg decreases from the clearance La to a clearance Lb.

A tooth Zb2 of the gear part 21cg is disposed at a position where a clearance Lz between the tooth Zb2 and an addendum circle (dotted line) of the tooth part 29g of the internal gear 29 is present. Therefore, the internal gear 29 can rotate without interfering with the tooth Zb2. Meanwhile, the coupling part 21c is kept in the same state (unlocking state) as the state illustrated in Fig. 10.

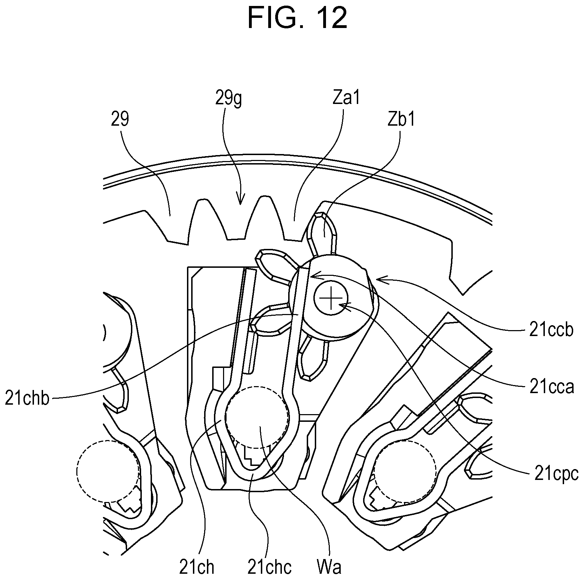

When the operation section 400 is rotated from the state illustrated in Fig. 11 in the locking direction, the internal gear 29 further rotates clockwise. The states of the internal gear 29 and the coupling part 21c at that time are illustrated in Fig. 12.

Fig. 12 is a diagram illustrating the states of the internal gear 29 and the coupling part 21c when the operation section 400 is rotated from the release position in the locking direction.

As illustrated in Fig. 12, when the operation section 400 is rotated from the release position in the locking direction, the tooth Za1 of the internal gear 29 and the tooth Zb1 of the gear part 21cg come into contact with each other. Meanwhile, the coupling part 21c is in the same state as the state illustrated in Figs. 10 and 11 and is kept in the unlocking state.

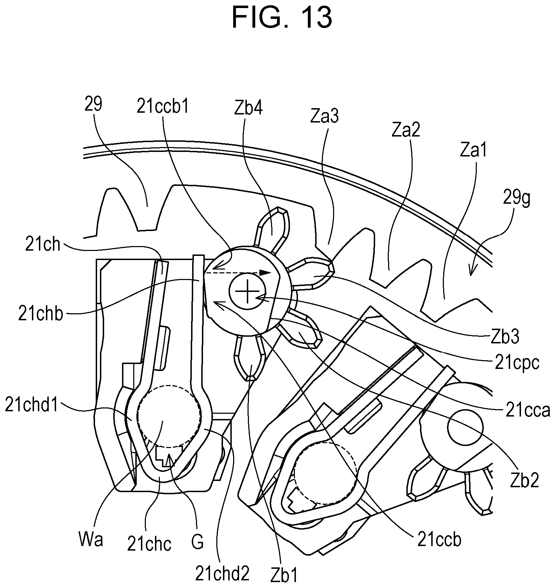

Fig. 13 is a diagram illustrating a state in which the pressing member 21cp rotates as the operation section 400 rotates in the locking direction.

As illustrated in Fig. 13, when the operation section 400 is further rotated from the state illustrated in Fig. 12 in the locking direction, the internal gear 29 further rotates clockwise.

The internal gear 29 rotates the gear part 21cg clockwise as the internal gear 29 moves from the state illustrated in Fig. 12 to the state illustrated in Fig. 13. When the gear part 21cg rotates, the holding surface 21cca is separated from the part 21chb to be pressed and the pressing surface 21ccb approaches the part 21chb to be pressed. Then, the first portion 21chd1 and the second portion 21chd2 start sandwiching the part Wa to be held.

Then, a tooth Za3 of the internal gear 29 moves to a position where the tooth Za3 is separated from a tooth Zb3 of the gear part 21cg while the part 21chb to be pressed is pressed by a corner part 21ccb1 disposed at an end of the pressing surface 21ccb. In this case, the part Wa to be held is sandwiched by the first portion 21chd1 and the second portion 21chd2.

When the tooth Za3 of the internal gear 29 is separated from the tooth Zb3 of the gear part 21cg, the transmission of a drive force from the internal gear 29 to the gear part 21cg ends. In this case, the cam 21cc is in a state in which the corner part 21ccb1 receives a reaction force from the plate spring 21ch.

The reaction force of the plate spring 21ch that acts on the corner part 21ccb1 acts on a position separate from the center 21cpc of the rotation of the pressing member 21cp in a radial direction of the rotation of the pressing member 21cp, and the pressing member 21cp rotates clockwise. In this case, the pressing member 21cp rotates in the same direction as a direction in which the pressing member 21cp is rotated by the internal gear 29 rotating clockwise.

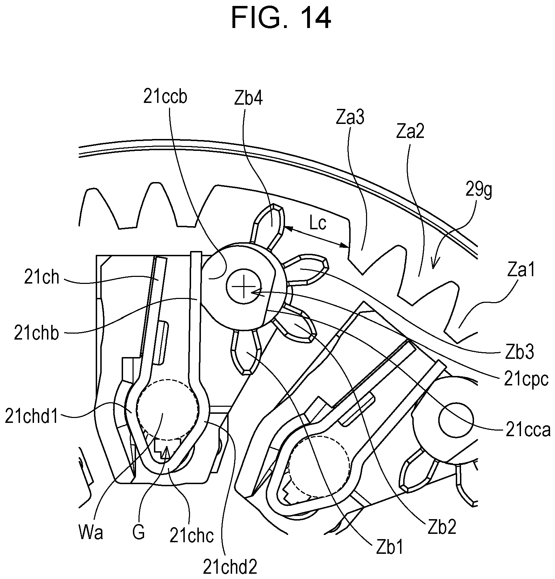

Fig. 14 is a diagram illustrating states of the internal gear 29 and the coupling part 21c in a state in which the operation section 400 is present at the fixation position.

As illustrated in Fig. 14, the pressing member 21cp receives the reaction force of the plate spring 21ch and further rotates from the state illustrated in Fig. 13.

As illustrated in Fig. 14, the pressing member 21cp stops in a state in which the pressing surface 21ccb of the cam 21cc and the part 21chb that is to be pressed and is included in the plate spring 21ch are in surface contact with each other. That is, the pressing surface 21ccb and the surface of the part 21chb to be pressed are arranged side by side on the same plane.

In this case, the coupling part 21c is in the locking state. When the coupling part 21c is in the locking state, the cam part 21cc of the pressing member 21cp is located at the pressing position, and the pressing surface 21ccb presses the part 21chb to be pressed.

When the coupling part 21c is in the locking state, the part Wa to be held is sandwiched by the first portion 21chd1 and the second portion 21chd2. That is, the plate spring 21ch is pressed by the cam 21cc and the part Wa to be held is tightened by the plate spring 21ch. As a result, the part Wa to be held is fixed by the plate spring 21ch.

After the catheter unit 100 is attached to the base unit 200 in the above-described manner, when the operation section 400 is moved to the fixation position, the catheter unit 100 enters a fully attached state.

In the present Example, at a position where the first portion 21chd1 and the second portion 21chd2 are separated from each other, the plate spring 21ch presses the part Wa to be held. Further, a flexed part 21chc connecting the first portion 21chd1 and the second portion 21chd2 is disposed between the first portion 21chd1 and the second portion 21chd2. The flexed part 21chc is disposed with a gap G between the flexed part 21chc and the part Wa to be held. In this manner, the first portion 21chd1 and the second portion 21chd2 can stably fix the part Wa to be held.

When the coupling part 21c is in the locking state, pulling the part Wa to be held from between the first portion 21chd1 and the second portion 21chd2 is restricted.

The tooth Za3 of the internal gear 29 and a tooth Zb4 of the gear part 21cg are stopped at positions between which a clearance Lc occurs.

To release the fixation of the drive wire W to the coupling part 21c, the operation section 400 at the fixation position is rotated in the release direction. In this case, the internal gear 29 rotates counterclockwise from the state illustrated in Fig. 14. When the internal gear 29 rotates counterclockwise, the tooth Za3 of the internal gear 29 comes into contact with the tooth Zb4 of the gear part 21cg, and the pressing member 21cp is rotated counterclockwise.

As the internal gear 29 is rotated counterclockwise, the fixation of the drive wire W by the coupling part 21c is released. The operations of the internal gear 29 and the operation of the pressing member 21cp in this case are opposite to the operations described above. That is, the fixation of the drive wire W by the coupling part 21c is released by an operation opposite to the above-described operation for fixing the drive wire W by the coupling part 21c.

The above-described operation is performed by the first to ninth coupling parts (21c11 to 21c33). That is, in a process of moving the operation section 400 from the detachment position to the fixation position, the first to ninth coupling parts (21c11 to 21c33) change from the unlocking state to the locking state due to the movement (rotation) of the operation section 400. In a process of moving the operation section 400 from the fixation position to the detachment position, the first to ninth coupling parts (21c11 to 21c33) change from the locking state to the unlocking state due to the movement (rotation) of the operation section 400. As described above, it is possible to switch between the unlocking state and the locking state of the first to ninth coupling parts (21c11 to 21c33) by the operation of rotating the single operation section 400.

That is, it is not necessary that an operation section for switching between the unlocking state and the locking state be provided for each of the plurality of coupling parts 21c and operated by the user. Therefore, the user can easily attach and detach the catheter unit 100 to and from the base unit 200.

A state in which each of the first to ninth drive wires (W11 to W33) is fixed by each of the first to ninth coupling parts (21c11 to 21c33) is referred to as a first state. A state in which the fixation of each of the first to ninth drive wires (W11 to W33) by each of the first to ninth coupling parts (21c11 to 21c33) is released is referred to as a second state.

The first state and the second state are switched in coordination with the movement of the operation section 400. That is, the first state and the second state are switched in coordination with the movement of the operation section 400 between the detachment position and the fixation position.

The internal gear 29 is configured to operate in coordination with the operation section 400. In the present Example, the joint 28 functions as a transmission member for interlocking the operation section 400 with the internal gear 29. The internal gear 29 and the joint 28 function as an interlocking part that interlocks with the operation section 400 such that the first state and the second state are switched in coordination with the movement of the operation section 400.

Specifically, the internal gear 29 and the joint 28 move, in coordination with the movement of the operation section 400, the part (part 21chb to be pressed) of the plate spring 21ch with respect to the part Wa to be held in a state in which the catheter unit 100 is attached to the base unit 200. The locking state and the unlocking state of the coupling part 21c are switched by the movement of the part 21chb to be held.

### <Insertion of Catheter into Human Body>

The medical device 1 is assumed to be inserted into the target, for example, the bronchus or the like. The catheter 11 is inserted into a long narrow space and moved in the long narrow space. To insert the catheter 11 into the target, there are a method in which the user holds the medical device 1 to insert the catheter 11 and a method in which the medical device 1 is attached to the moving stage 2a of the support base 2 and the moving motor 31 within the support base 2 is driven to slide the moving stage 2a.

The shape of the organ such as the bronchus is complex and it is necessary to smoothly move the catheter 11 without applying an unnecessary force to the inner wall of the organ.

As control for smoothly moving the catheter 11, Follow The Leader control is provided. The Follow The Leader control is to cause the succeeding second guide ring J2 and the succeeding first guide ring J1 to pass through a path through which the third guide ring J3 at the most distal end side of the catheter 11 has passed during the insertion in the target. The Follow The Leader control is implemented by transferring the control of the third guide ring J3 at the head to the control of the succeeding second guide ring J2 and the control of the succeeding first guide ring J1 at different times. The different times are determined based on the speed at which the catheter 11 is inserted and spacing between the guide rings J1 to J3. The catheter 11 can be smoothly moved by the Follow The Leader control even in a case where the path is a long and thin path having a complex shape, such as the bronchus. The third guide ring J3 that is on the most distal end side of the catheter 11 is operated by the user using the input device 3b of the control apparatus 3.

Meanwhile, the bronchus or the like is not necessarily an ideal arc-shaped path, and the inner diameter of the path is not fixed. In addition, when the user operates the catheter 11 using the input device 3b, the number of human errors cannot be reduced to zero. Therefore, in a state in which the catheter 11 is inserted in the target, the catheter 11 may come into contact with the inner wall of the organ and it may become difficult to move the catheter 11. In this state, a tensile force occurs in the drive wire W and can be detected by the force sensor 21cf. In a case where the tensile force detected by the force sensor 21cf exceeds a predetermined value, control is performed to move the drive wire W and change a bent shape of the catheter 11 such that the tensile force becomes equal to or less than the predetermined value, that is, the tensile force is reduced. This control is generally referred to as back drive control, but is referred to as back drive control of the drive source M in the present Example. By the back drive control of the drive source M, the catheter 11 can have a shape along the path within the target, and can be smoothly moved without applying an excessive force to the inner wall of the organ.

The catheter 11 is inserted into the target while the Follow The Leader control and the back drive control described above are used. Both the Follow The Leader control and the back drive control are performed on the drive source M that changes the bent shape of the catheter 11.

However, in a case where the moving motor 31 within the support base 2 is driven to slide the moving stage 2a, and the catheter 11 is inserted into the target, it may be difficult to insert the catheter 11 due to the movement. For example, it is conceivable that a state in which the distal end that is the tip of the catheter 11 comes into contact with the inner wall of the organ from a substantially front side is caused by the movement of the catheter 11 by the moving motor 31 in the insertion direction. In this state, the moving motor 31 needs to be driven in an opposite direction (direction in which the moving stage 2a is slid in a direction in which the catheter 11 is pulled out) to release the catheter 11 from the contact and reduce the tensile that has occurred in the drive wire W without controlling the drive source M to change the bent shape of the catheter 11.

In the present Example, in a case where the catheter 11 comes into contact with the inner wall of the organ in a state in which the moving motor 31 within the support base 2 is driven, and it is estimated that it is difficult to further insert the catheter 11, this situation is eliminated by controlling the moving motor 31. Specifically, in a case where the moving stage 2a is slid by driving the moving motor 31, and the catheter 11 is inserted in the target, when tensile forces detected by force sensors (hereinafter denoted by 21cf31 to 21cf33 for convenience) connected to the seventh to ninth drive wires W31 to W33 connected to the third guide ring J3 on the most distal end side are all tensile forces in the same direction and at least one of the tensile forces detected by the force sensors 21cf31 to 21cf33 exceeds the predetermined value, the moving motor 31 is driven in the opposite direction to slide the moving stage 2a in the direction in which the catheter 11 is pulled out. It is determined whether or not the tensile forces in the drive wires W31 to W33 extending to the most distal end side of the catheter 11 are all tensile forces in the same direction and whether or not any one of the tensile forces exceeds the predetermined value. This is due to the fact that, in a case where the tensile forces detected by the force sensors 21cf31 to 21cf33 are all tensile forces in the same direction and at least one of the tensile forces detected by the force sensors 21cf31 to 21cf33 exceeds the predetermined value, it is estimated that the catheter 11 comes into contact with the inner wall of the organ and that it is difficult to further insert the catheter 11. This control is referred to as back drive control of the moving motor 31 in the present Example.

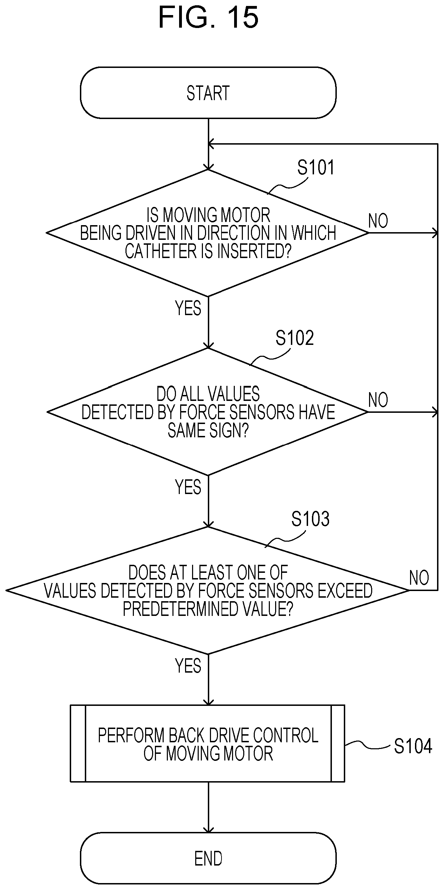

A control process to be performed by the control apparatus 3 for releasing from contact will be described with reference to Fig. 15. Fig. 15 is a flowchart illustrating the control process to be performed by the control apparatus 3 for releasing from contact.

In step S101, the control apparatus 3 determines whether or not the moving motor 31 is being driven in the direction in which the catheter 11 is inserted into the target. In a case where the moving motor 31 is being driven in the direction in which the catheter 11 is inserted (YES in step S101), the process proceeds to step S102. In a case where the moving motor 31 is not being driven in the direction in which the catheter 11 is inserted (NO in step S101), the determination in step S101 is repeated.

In step S102, the control apparatus 3 determines whether or not all of values detected by the force sensors 21cf31 to 21cf33 connected to the seventh to ninth drive wires W31 to W33 connected to the third guide ring J3 on the most distal end side have the same sign, that is, whether or not tensile forces detected by the force sensors 21cf31 to 21cf33 are all tensile forces in the same direction. In a case where all of the values detected by the force sensors 21cf31 to 21cf33 have the same sign (YES in step S102), the process proceeds to step S103. In a case where not all of the values detected by the force sensors 21cf31 to 21cf33 have the same sign (No in step S102), the process returns to step S101.

In step S103, the control apparatus 3 determines whether or not at least one of the values detected by the force sensors 21cf31 to 21cf33 exceeds the predetermined value, that is, whether or not at least one of the tensile forces determined to be in the same direction in step S102 exceeds the predetermined value. In a case where at least one of the values detected by the force sensors 21cf31 to 21cf33 exceeds the predetermined value (YES in step S103), the process proceeds to step S104. In a case where the values detected by the force sensors 21cf31 to 21cf33 do not exceed the predetermined value (NO in step S103), the process returns to step S101.

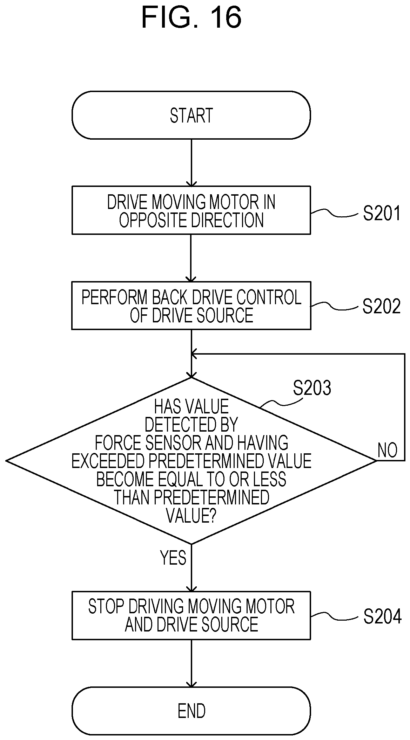

In step S104, since it is estimated that the catheter 11 comes into contact with the inner wall of the organ and that it is difficult to further insert the catheter 11, the control apparatus 3 performs the back drive control of the moving motor 31 and thereafter ends the process. As described above, the back drive control of the moving motor 31 is control for driving the moving motor 31 in the opposite direction to slide the moving stage 2a in the direction in which the catheter 11 is pulled out, and details of the back drive control of the moving motor 31 will be described with reference to Fig. 16.

Next, the back drive control of the moving motor 31 in step S104 illustrated in Fig. 15 will be described with reference to Fig. 16. Fig. 16 is a flowchart illustrating a process of the back draft control of the moving motor 31.

In step S201, the control apparatus 3 drives the moving motor 31 in the opposite direction. Therefore, it is possible to release the catheter 11 from the contact by sliding the moving stage 2a in which the catheter 11 is pulled out.

In step S202, the control apparatus 3 performs the back drive control of the drive source M on the drive source M within the base unit 200. As described above, the back drive control of the drive source M is control to detect, by the force sensor 21cf, a tensile force that has occurred in the drive wire W, and move the drive wire W to change a bent shape of the catheter 11 such that, in a case where the tensile force detected by the force sensor 21fc exceeds the predetermined value, the tensile force becomes equal to or less than the predetermined value, that is, the tensile force is reduced. Therefore, it is possible to change the shape of the catheter 11 to a shape along the path within the target, and smoothly move the catheter 11 without applying an excessive force to the inner wall of the organ.

In step S203, the control apparatus 3 determines whether or not the value detected by the force sensor 21cf and having exceeded the predetermined value in step S103 illustrated in Fig. 15 has become equal to or less than the predetermined value. In a case where the value detected by the force sensor 21cf has become equal to or less than the predetermined value (YES in step S203), the process proceeds to step S204. In a case where the value detected by the force sensor 21cf has not become equal to or less than the predetermined value (NO in step S203), the determination in step S203 is repeated. As described above, in a case where the value detected by the force sensor 21cf and having exceeded the predetermined value in step S103 illustrated in Fig. 15 exceeds the predetermined value, the value detected by the force sensor 21cf is continuously monitored, and the moving motor 31 and the drive source M are continuously driven until the value detected by the force sensor 21cf becomes equal to or less than the predetermined value.

In step S204, the control apparatus 3 stops driving the moving motor 31 and the drive source M and ends the process.

As described above, in a case where the catheter 11 comes into contact with the inner wall of the organ and it is estimated that it is difficult to further insert the catheter 11, it is possible to release the catheter 11 from the contact by driving the moving motor 31 in the opposite direction to slide the moving stage 2a in the direction in which the catheter 11 is pulled out. Then, by performing the back drive control of the drive source M at that time, it is possible to change the shape of the catheter 11 to a shape along the path within the target and to smoothly move the catheter 11 without applying an excessive force to the inner wall of the organ.

### [Example 2]

Next, Example 2 will be described. Basic configurations and processing operations of the medical system 1A and the medical device 1 are similar to those described in Example 1. Constituent elements common to Example 1 are denoted by the same reference signs, description thereof is omitted, and differences from Example 1 will be mainly described.

As described in Example 1, the Follow The Leader control is provided as control for smoothly moving the catheter 11. The Follow The Leader control is performed such that as the catheter 11 is inserted into the target, the succeeding parts pass through the same path as a path through which the preceding part has passed.

That is, every time the catheter 11 is moved by the moving motor 31, a bent shape of the preceding part is stored. Then, when the catheter 11 is further moved, the succeeding parts reproduce the bent shape of the preceding part stored at an insertion position. The insertion position where the catheter 11 is inserted in the target is an amount by which the moving stage 2a on which the medical device 1 is placed is slid. The amount by which the moving stage 2a is slid is an amount by which the moving motor 31 is driven. The amount by which the moving motor 31 is driven can be calculated by counting the number of drive pulses of the moving motor 31 or providing an encoder or the like and detecting a rotation amount of the moving motor 31. In addition, the bent shape of the catheter 11 is determined based on an amount by which the drive wire W is moved. The amount by which the drive wire W is moved is an amount by which the drive source M is driven. The amount by which the drive source M is driven can be detected by an encoder disposed in the drive source M. Storing the amount by which the drive source M is driven for each insertion position of the catheter 11 means storing a bent shape of the catheter 11 in time series. As the insertion position of the catheter 11 is advanced by the moving motor 31, the succeeding parts (second guide ring J2 and first guide ring J1) reach the stored insertion position. The succeeding parts can reproduce the bent shape of the preceding part at the same insertion position by reading amounts by which the drive sources M are driven for the preceding parts (the third guide ring J3 for the second guide ring J2, and the second guide ring J2 for the first guide ring J1) and that have been stored together with the insertion position, and driving the drive sources M by the same amounts as the read drive amounts. By continuously performing this, the Follow The Leader control is implemented.

Meanwhile, when the moving motor 31 is driven in the opposite direction to slide the moving stage 2a in the direction in which the catheter 11 is pulled out, it is necessary to smoothly move the catheter 11. In this case, when the drive source M is driven by the same amount as the amount by which the drive source M has been driven at the past same insertion position, it is possible to reproduce a bent shape of the catheter 11 at that time. Therefore, the shape of each of the parts of the catheter 11 can be changed to a bent shape that enables the part to pass through the same path as a path through which a part among the parts of the catheter 11 has already passed, and it is possible to smoothly pull the catheter 11 out of the inside of the target. This is referred to as Follow The Leader control for reverse rotation.

In Example 2, as illustrated in Fig. 15, the control apparatus 3 performs a control process for releasing from contact. In this case, the Follow The Leader control for reverse rotation is performed when the back drive control of the moving motor 31 in step S104 is performed. That is, a bent shape of the catheter 11 is controlled to be the same as a bent shape at the same insertion position stored in a memory that is a storage unit.

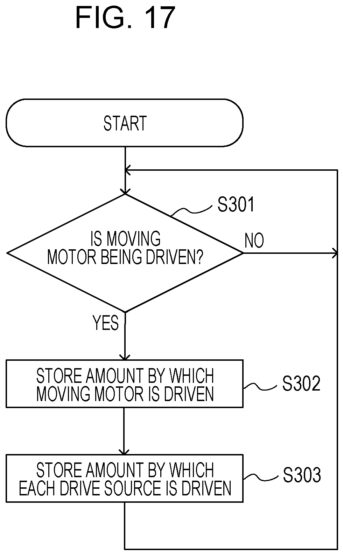

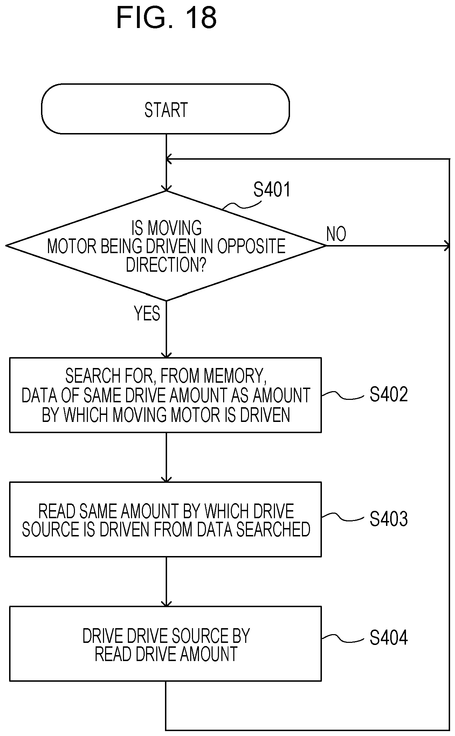

First, the Follow The Leader control for reverse rotation will be described with reference to Figs. 17 and 18.

Fig. 17 is a flowchart illustrating a data acquisition process for the Follow The Leader control.

In step S301, the control apparatus 3 determines whether or not the moving motor 31 is being driven. In a case where the moving motor 31 is being driven (YES in step S301), the process proceeds to step S302. In a case where the moving motor 31 is not being driven (NO in step S301), the determination in step S301 is repeated.

In step S302, the control apparatus 3 stores, to the memory, an amount by which the moving motor 31 is driven and that is information indicating the insertion position of the catheter 11.

In step S303, the control apparatus 3 stores, to the memory, an amount by which the drive source M is driven and that is information indicating a bent shape of the catheter 11, in association with the amount by which the moving motor 31 is driven and that has been stored in step 302. Then, the process returns to step S301 again.

By performing the data acquisition process each time the moving motor 31 is driven, data in which an insertion position of the catheter 11 is associated with a bent shape of the catheter 11 is accumulated in the memory. The data is also used for the Follow The Leader control, and thus is constantly in operation while the medical device 1 is in use.

Fig. 18 is a flowchart illustrating a process of the Follow The Leader control for reverse rotation.

In step S401, the control apparatus 3 determines whether or not the moving motor 31 is being driven in the opposite direction. In a case where the moving motor 31 is being driven in the opposite direction (YES in step S401), the process proceeds to step S402. In a case where the moving motor 31 is not being driven in the opposite direction (NO in step S401), the determination in step S401 is repeated.

In step S402, the control apparatus 3 searches for, from the memory, the same drive amount as an amount by which the drive motor 31 is driven in the opposite direction in step S401, that is, past data of the same insertion position.

In step S403, the control apparatus 3 reads an amount by which the drive source M is driven and that is associated with the amount by which the drive motor 31 is driven and that has been searched in step S401.

In step S404, the control apparatus 3 drives the drive source M by the same amount as the amount by which the drive source M is driven and that has been read in step S403. Then, the process returns to step S401 again, and the same control is repeated for a time period for which the moving motor 31 is continuously driven in the opposite direction.

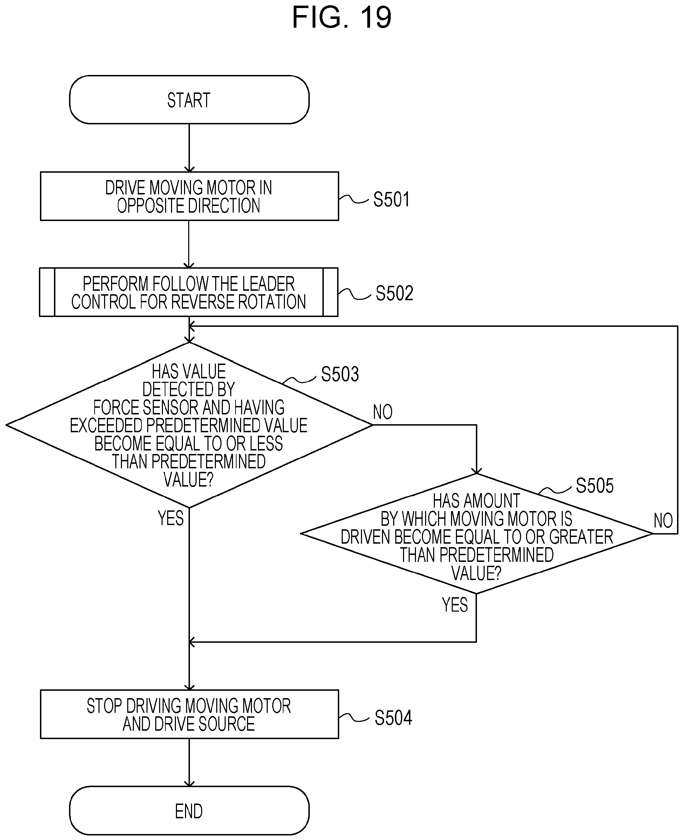

Next, the back drive control of the moving motor 31 in step S104 illustrated in Fig. 15 will be described with reference to Fig. 19. Fig. 19 is a flowchart illustrating a process of the back draft control of the moving motor 31.

In step S501, the control apparatus 3 drives the moving motor 31 in the opposite direction. Therefore, it is possible to slide the moving stage 2a in the direction in which the catheter 11 is pulled out, and release the catheter 11 from contact.

In step S502, the control apparatus 3 performs the Follow The Leader control for reverse rotation on the drive source M within the base unit 200. The Follow The Leader control for reverse rotation is described above with reference to Figs. 17 and 18. Therefore, it is possible to deform each of the parts of the catheter 11 into a bent shape that enables the part to pass through the same path as a path through which a part among the parts has already passed, and it is possible to smoothly pull the catheter 11 out of the inside of the target.

In step S503, the control apparatus 3 determines whether or not the value detected by the force sensor 21cf and having exceeded the predetermined value in step S103 illustrated in Fig. 15 has become equal to or less than the predetermined value. In a case where the value detected by the force sensor 21cf has become equal to or less than the predetermined value (YES in step S503), the process proceeds to step S504. In a case where the value detected by the force sensor 21cf has not become equal to or less than the predetermined value (NO in step S503), the process proceeds to step S505.

In step S505, the control apparatus 3 determines whether or not the amount by which the moving motor 31 is driven has become equal to or greater than a predetermined value. In a case where the amount by which the moving motor 31 is driven has become equal to or greater than the predetermined value (YES in step S505), the process proceeds to step S504. In a case where the amount by which the moving motor 31 is driven has not become equal to or greater than the predetermined value (NO in step S505), the process returns to step S503. For example, the patient may move or the like, and the shape of the path within the target may change. In this case, the catheter 11 may have a bent shape before the change by performing the Follow The Leader control for reverse rotation, a tensile force that has occurred in the drive wire W may not be reduced, and the moving motor 31 may be continuously driven in the opposite direction. Therefore, after the back drive control of the moving motor 31 is started, in a case where the value detected by the force sensor 21cf does not become equal to or less than the predetermined value (NO in step S503), the control apparatus 3 determines whether or not the amount by which the moving motor 31 is driven has become equal to or greater than the predetermined value as in this step. In a case where the amount by which the moving motor 31 is driven has become equal to or greater than the predetermined value, the control apparatus 3 determines that the shape of the path within the target has changed for some reason, the bent shape of the catheter 11 is not along the shape of the path, and the tensile force that has occurred in the drive wire W is not reduced, and the process proceeds to step S504.

In step S504, the control apparatus 3 stops driving the moving motor 31 and the drive source M and ends the process.

As described above, in a case where the catheter 11 comes into contact with the inner wall of the organ and it is estimated that it is difficult to further insert the catheter 11, the moving motor 31 is driven in the opposite direction to slide the moving stage 2a in a direction in which the catheter 11 is pulled out and thus the catheter 11 can be easily released from the contact. Then, by performing the Follow The Leader control for reverse rotation at that time, each of the parts of the catheter 11 can be deformed into a bent shape that enables the part to pass through the same path as a path through which a part among the parts of the catheter 11 has already passed. Therefore, the catheter 11 can be smoothly pulled out of the inside of the target.

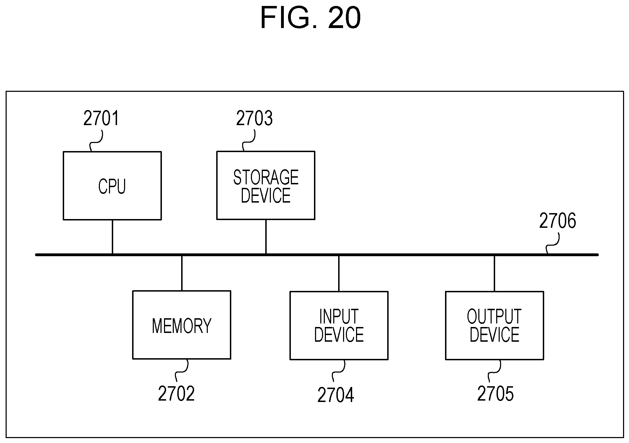

Fig. 20 illustrates an example of a hardware configuration for implementing the control means of the present invention. As the hardware configuration for implementing the control means of the present invention, a CPU 2701, a memory 2702, a storage device 2703, an input device 2704, and an output device 2705 are provided and connected to each other via a bus 2706.

The CPU 2701 executes a program stored in the storage device 2703. As a result, the processes in the flowcharts described above are performed. As the CPU 2701 executes the program, functions of acquisition means, notification means, and control prohibition means according to the present invention are implemented. The memory 2702 temporarily stores the program and data read by the CPU 2701 from the storage device 2703. In addition, the memory 2702 is also used as a region for the CPU 2701 to execute various programs. The storage device 2703 stores an operating system (OS), various programs, and various data. The input device 2704 is a functional unit that receives input from the user, and for example, a keyboard and a mouse are used. The output device 2705 outputs information input by the input device 2704 and results of executing the programs by the CPU 2701.

Although the present invention is described above together with the embodiments, the above-described embodiments are merely examples of implementation of the present invention, and the technical scope of the present invention should not be construed to be limited by these embodiments. That is, the present invention can be implemented in various forms without departing from the technical idea thereof or the main features thereof.

### (Other Embodiments)

The present invention can be implemented by supplying a program that implements one or more of the functions described in the embodiments to a system or an apparatus via a network or a storage medium, and reading and executing the program by one or more processors in a computer of the system or the apparatus. In addition, the present invention can also be implemented by a circuit (for example, an ASIC) that implements one or more of the functions.

The present invention is not limited to the above-described embodiments and can be variously changed and modified without departing from the spirit and scope of the present invention. Therefore, the following claims are appended to set forth the scope of the present invention.

This application claims priority from Japanese Patent Application No. 2022-028144 filed on February 25, 2022, which is hereby incorporated by reference herein in its entirety.

### Reference Signs List

1 medical device
1A medical system
2 support base
2a moving stage
3 control apparatus
11 catheter
21cf force sensor
31 moving motor
100 catheter unit
200 base unit
M drive source
W drive wire

## Claims

1. A continuum robot system comprising:
a continuum robot including a bendable body configured to bend via a plurality of linear members, a first drive source that moves the linear members, and a tensile force detector that detects tensile forces that have occurred in the linear members;
a support base including a moving stage to which the continuum robot is attached, and a second drive source that slides the moving stage; and
control means that controls, in a case where the tensile forces of the plurality of linear members detected by the tensile force detector are tensile forces in a same direction and at least one of the tensile forces exceeds a predetermined value when the second drive source is driven in a direction in which the bendable body is inserted into a target, the second drive source such that the second drive source is stopped or driven in an opposite direction.

2. The continuum robot system according to claim 1, wherein
the control means controls the second drive source such that the second drive source is driven in the opposite direction so as to change a bent shape of the bendable body based on the tensile forces of the linear members detected by the tensile force detector.

3. The continuum robot system according to claim 1, further comprising a storage unit that stores information indicating an insertion position of the bendable body, and information indicating a bent shape of the bendable body, wherein
the control means controls the second drive source such that the second drive source is driven in the opposite direction so as to control, according to the insertion position of the bendable body, the bent shape of the bendable body to be identical to a bent shape at the same insertion position stored in the storage unit.

4. The continuum robot system according to any one of claims 1 to 3, wherein
the control means controls the second drive source such that the second drive source is driven in the opposite direction, and stops the second drive source when the tensile force that has exceeded the predetermined value becomes equal to or less than the predetermined value.

5. The continuum robot system according to any one of claims 1 to 4, wherein
the control means controls the second drive source such that the second drive source is driven in the opposite direction, and stops the second drive source when an amount by which the second drive source is driven reaches a predetermined value.

6. The continuum robot system according to any one of claims 1 to 5, wherein
the control means determines whether or not the tensile forces of the plurality of linear members detected by the tensile force detector for the plurality of linear members extending to a most distal end side of the bendable body are tensile forces in a same direction, and whether or not at least one of the tensile forces exceeds a predetermined value.

7. A method for controlling a continuum robot system including: a continuum robot including a bendable body configured to bend via a plurality of linear members, a first drive source that moves the linear members, and a tensile force detector that detects tensile forces that have occurred in the linear members; and a support base including a moving stage to which the continuum robot is attached, and a second drive source that slides the moving stage, the method comprising a step of controlling, in a case where the tensile forces of the plurality of linear members detected by the tensile force detector are tensile forces in a same direction and at least one of the tensile forces exceeds a predetermined value when the second drive source is driven in a direction in which the bendable body is inserted into a target, the second drive source such that the second drive source is stopped or driven in an opposite direction.

8. A program for controlling a continuum robot system including: a continuum robot including a bendable body configured to bend via a plurality of linear members, a first drive source that moves the linear members, and a tensile force detector that detects tensile forces that have occurred in the linear members; and a support base including a moving stage to which the continuum robot is attached, and a second drive source that slides the moving stage, the program causing a computer to execute processing of controlling, in a case where the tensile forces of the plurality of linear members detected by the tensile force detector are tensile forces in a same direction and at least one of the tensile forces exceeds a predetermined value when the second drive source is driven in a direction in which the bendable body is inserted into a target, the second drive source such that the second drive source is stopped or driven in an opposite direction.
